(19) Europäisches Patentamt
European Patent Office
Office européen des brevets

(11) EP 1 102 746 B1

(12) **EUROPÄISCHE PATENTSCHRIFT**

(45) Veröffentlichungstag und Bekanntmachung des Hinweises auf die Patenterteilung:
**14.05.2003 Patentblatt 2003/20**

(21) Anmeldenummer: **99938296.3**

(22) Anmeldetag: **20.07.1999**

(51) Int Cl.7: **C07D 211/34**, A61K 31/445

(86) Internationale Anmeldenummer:
**PCT/EP99/05161**

(87) Internationale Veröffentlichungsnummer:
**WO 00/005207 (03.02.2000 Gazette 2000/05)**

(54) **SUBSTITUIERTE PHENYLAMIDINE MIT ANTITHROMBOTISCHER WIRKUNG**

SUBSTITUTED PHENYLAMIDINES WITH ANTITHROMBOTIC ACTION

PHENYLAMIDINES SUBSTITUEES A EFFET ANTITHROMBOTIQUE

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **23.07.1998 DE 19833105**

(43) Veröffentlichungstag der Anmeldung:
**30.05.2001 Patentblatt 2001/22**

(73) Patentinhaber: **Boehringer Ingelheim Pharma GmbH & Co.KG**
**55218 Ingelheim am Rhein (DE)**

(72) Erfinder:
- **HIMMELSBACH, Frank**
  **D-88441 Mittelbiberach (DE)**
- **GUTH, Brian**
  **D-88447 Warthausen (DE)**
- **SCHUBERT, Hans-Dieter**
  **D-88400 Biberach (DE)**

(74) Vertreter: **Hammann, Heinz, Dr.**
**c/o Boehringer Ingelheim GmbH,**
**CD Patents**
**55216 Ingelheim am Rhein (DE)**

(56) Entgegenhaltungen:
**EP-A- 0 805 149** **WO-A-96/33970**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 102 746 B1

Printed by Jouve, 75001 PARIS (FR)

## Beschreibung

Unter den Schutzumfang der WO 96/33970 fallen Phenylamidine der allgemeinen Formel

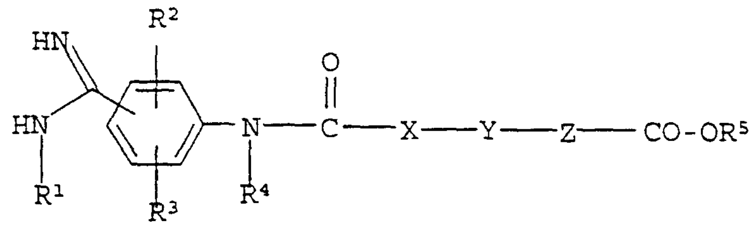

in der u. a. $R^1$ eine $C_{1-4}$-Alkyloxycarbonylgruppe, eine Aryl-$C_{1-3}$-alkyloxycarbonylgruppe oder eine Gruppe der Formel

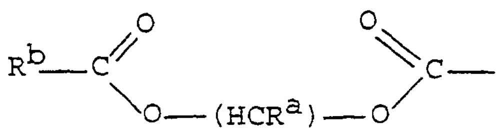

,

wobei
$R^a$ ein Wasserstoffatom oder eine Alkylgruppe und
$R^b$ eine Alkylgruppe oder eine 3- bis 7-gliedrige Cycloalkylgruppe darstellen, bedeutet, wobei keine derartige Verbindung expressis verbis in dieser Offenlegungsschrift beschrieben wird.

EP 0 805 149 A1 beschreibt 2,3-Diketopiperazinderivate mit antithrombotischer Wirkung.

Es wurde nun gefunden, daß die Phenylamidine der allgemeinen Formel

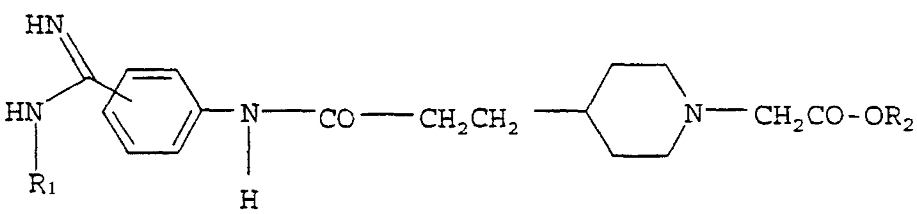

(I),

in der
$R_1$ eine $C_{5-12}$-Alkyloxycarbonylgruppe
$R_7$ eine $C_{1-4}$-Alkyl- oder $C_{5-6}$-Cycloalkylgruppe
bedeuten, deren Tautomere, deren Stereoisomere und deren Salze, insbesondere deren physiologisch verträgliche Salze mit anorganischen oder organischen Säuren oder Basen, noch wertvollere pharmakologische Eigenschaften aufweisen, vorzugsweise antithrombotische Wirkungen.

Gegenstand der vorliegenden Erfindung sind die Verbindungen der obigen allgemeinen Formel I, deren Tautomere, deren Stereoisomere und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Bevorzugte Verbindungen der obigen allgemeinen Formel I sind diejenigen, in denen
die substituierte Amidinogruppe in 4-Stellung steht,
deren Tautomere, deren Stereoisomere und deren Salze.

Als besonders bevorzugte Verbindungen seien beispielsweise folgende erwähnt:

(1) 4-[2-[[4-(Octyloxycarbonylamidino)phenyl]aminocarbonyl]-ethyl]-1-[(ethoxycarbonyl)methyl]-piperidin,

(2) 4-[2-[[4-(Hexyloxycarbonylamidino)phenyl]aminocarbonyl]-ethyl]-1-[(ethoxycarbonyl)methyl]-piperidin,

(3) 4-[2-[[4-(Hexyloxycarbonylamidino)phenyl]aminocarbonyl]ethyl]-1-[(methoxycarbonyl)methyl]-piperidin und

(4) 4-[2-[[4-(Octyloxycarbonylamidino)phenyl] aminocarbonyl]ethyl]-1-[(methoxycarbonyl)methyl]-piperidin

sowie deren Salze.

Erfindungsgemäß erhält man die neuen Verbindungen der allgemeinen Formel I beispielsweise nach folgendem Verfahren:

Umsetzung einer Verbindung der allgemeinen Formel

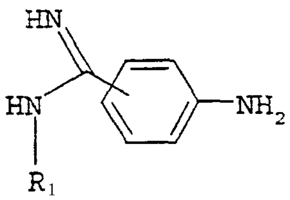

(II),

in der

$R_1$ wie eingangs definiert ist, mit einer Verbindung der allgemeinen Formel

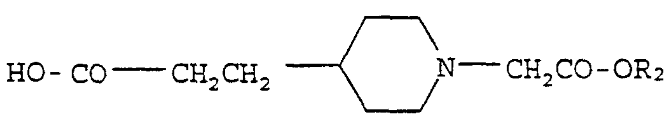

(III),

in der

$R_2$ wie eingangs definiert ist, oder deren reaktionsfähigen Derivaten und

gegebenenfalls anschließende Überführung des Restes $R_7$ in ein Wasserstoffatom.

Als reaktionsfähige Derivate einer Verbindung der allgemeinen Formel III kommen beispielsweise deren Säurechloride, Säureazide, gemischte Anhydride mit aliphatischen oder aromatischen Carbonsäuren oder Kohlensäuremonoester, deren Imidazolide und deren Ester wie deren Alkyl-, Aryl- und Aralkylester wie der Methyl-, Ethyl-, Isopropyl-, Pentyl-, Phenyl-, Nitrophenyl- oder Benzylester in Betracht.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Pyridin, Pyridin/Methylenchlorid, Pyridin/Dimethylformamid, Benzol/Tetrahydrofuran oder Dioxan gegebenenfalls in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodimid, N,N'-Dicyclohexylcarbodiimid/N-Hydroxysuccinimid, 2-(1H-Benztriazolyl)-1,1,3,3-tetramethyl-uronium-Salzen, N,N'-Carbonyldiimidazol, N,N'-Thionyldiimidazol, 2-Chlor-1-methylpyridiniumjodid oder Triphenylphosphin/Tetrachlorkohlenstoff, gegebenenfalls in Gegenwart von Dimethylaminopyridin oder 1-Hydroxy-benztriazol und/oder einer Base wie Triethylamin, N-Ethyl-diisopropylamin, Pyridin oder N-Methyl-morpholin, zweckmäßigerweise bei Temperaturen zwischen -10 und 180°C, vorzugsweise bei Temperaturen zwischen 0 und 120°C, durchgeführt.

Die anschließende Überführung des Restes $R_2$ in ein Wasserstoffatom wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Ferner können die erhaltenen Verbindungen der allgemeinen Formel I, wie bereits eingangs erwähnt wurde, gegebenenfalls in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen sind teilweise literaturbekannt oder man erhält diese nach literaturbekannten Verfahren (siehe Beispiele).

Wie bereits eingangs erwähnt, weisen die neuen Phenylamidine der allgemeinen Formel I und deren Salze, insbesondere deren physiologisch verträgliche Salze mit anorganischen oder organischen Säuren oder Basen, wertvolle Eigenschaften auf. So werden mit den neuen Verbindungen der allgemeinen Formel I nach oraler Gabe hohe und langanhaltende Plasmaspiegel im Vergleich zu der in der WO 96/33970 beschriebenen aggregationshemmenden Verbindung 4-[2-[(4-Amidinophenyl)aminocarbonyl]ethyl]-1-carboxymethyl-piperidin (Verbindung A) erzielt. Somit weisen die neuen Phenylamidine der allgemeinen Formel I und deren Salze wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurde die Konzentration der Verbindung A nach oraler Gabe der Verbindungen der Beispiele 1 (Verbindung B) und 1(1) (Verbindung C) der vorliegenden Erfindung im Plasma bestimmt und mit der Plasmakonzentration der Verbindung A nach oraler Gabe der Verbindung A verglichen.

Nach oraler Gabe von 1 mg/kg der zu untersuchenden Verbindungen am Rhesusaffen wird die Konzentration der Verbindung A im Plasma 4, 8, 12 und 24 Stunden nach Substanzgabe gemessen. Hierzu wird das Rhesusplasma mit einer Suspension von Humanthrombozyten in Plasma und $^{3}$H-BIBU 52 (siehe DE-A-4,214,245) als Ligand inkubiert. Der freie und gebundene Ligand wird durch Zentrifugation getrennt und durch Szintillationszählung quantitativ bestimmt. Aus der Menge des gebundenen Liganden wird anhand einer Eichkurve die Konzentration der Verbindung A errechnet.

Dazu wird aus einer Antikubitalvene Spenderblut entnommen und mit Trinatriumzitrat antikoaguliert (Endkonzentration: 13 mMol/l). Das Blut wird 10 Minuten bei 170 x g zentrifugiert und das überstehende plättchenreiche Plasma (PRP) abgenommen. Das Restblut wird noch einmal bei 3200 x g scharf abzentrifugiert und das überstehende plättchenarme Plasma (PPP) abgenommen.

Für die Eichkurve zur Berechnung der Konzentration wird zu 995 µl PPP 5 µl Lösung der Verbindung A zugesetzt (Endkonzentration 5000 nMol/l). Aus dieser Plasmaprobe werden mit PPP weitere Plasmaproben verdünnt bis zu einer Endkonzentration von 2,5 nMol/l.

Zu 150 µl Plasmaprobe vom Rhesusaffen oder Eichkurvenplasma werden 10 µl $^{3}$H-BIBU 52 (Endkonzentration 10 nMol/l), 10 µl $^{14}$C-Sucrose (370 Bq) und 80 µl PRP gegeben und bei Raumtemperatur 20 Minuten inkubiert. Anschließend werden die Proben 5 Minuten bei 2000 x g zentrifugiert und der Überstand abgezogen. 100 µl des Überstandes werden versetzt mit 100 µl NaOH 0,2 Mol/l, 15 µl HCl 5 Mol/l und 2 ml Szintillator und die $^{3}$H- und $^{14}$C-Radioaktivität quantitativ gemessen. Das Pellet wird in 200 µl NaOH 0,2 Mol/l gelöst. 180 µl davon werden versetzt mit 15 µl HCl 5 Mol/l und 2 ml Szintillator und die $^{3}$H- und $^{14}$C-Radioaktivität gemessen. Das im Pellet noch verbliebene Restplasma wird aus dem $^{14}$C-Gehalt bestimmt und abgezogen. Die Menge des gebundenen Liganden wird aus dem $^{3}$H-Gehalt bestimmt. Die Menge des gebundenen Liganden wird gegen die Konzentration des Eichkurvenplasmas aufgetragen. Die Konzentration der Verbindung A im Rhesusplasma wird aus der Menge des gebundenen Liganden in der betreffenden Plasmaprobe im Vergleich mit der Eichkurve errechnet.

Die nachfolgende Tabelle enthält die gefundenen Werte:

| Substanz | Konz. von A in [nM], 4h | Konz. von A in [nM], 8h | Konz. von A in [nM], 12h | Konz. von A in [nM], 24h |
|---|---|---|---|---|
| A | 174 | 38 | 25 | 3 |
| B | 133 | 127 | 81 | 51 |
| C | 170 | 123 | 80 | 52 |

Auf Grund ihrer biologischen Eigenschaften eignen sich die neuen erfindungsgemäßen Verbindungen der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 μg und 30 mg/kg Körpergewicht, vorzugsweise bei 1 μg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, ADP-Rezeptorantagonisten, Clopidogrel, Ticlopidine, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

4-(Hexyloxycarbonylamidino)-anilin

5.1 g 4-(Hexyloxycarbonylamidino)-nitrobenzol werden in 100 ml Tetrahydrofuran in Gegenwart von 0.5 g Palladium auf Aktivkohle bei Raumtemperatur und einem Wasserstoffdruck von 50 psi hydriert. Anschließend wird vom Katalysator abgesaugt und das Filtrat eingeengt.
Ausbeute: 4.5 g 100 % der Theorie,
$R_f$-Wert: 0.23 (Kieselgel; Cyclohexan/Essigester = 1:1)

Analog Beispiel I werden folgende Verbindungen erhalten:

(1) 4-(Octyloxycarbonylamidino)-anilin
$R_f$-Wert: 0.25 (Kieselgel; Cyclohexan/Essigester = 1:1)
(2) 4-(Methoxycarbonylamidino)-anilin
$R_f$-Wert: 0.35 (Kieselgel; Methylenchlorid/Methanol = 9:1)=
(3) 4-(Benzyloxycarbonylamidino)-anilin
$R_f$-Wert: 0.35 (Kieselgel; Methylenchlorid/Methanol/konz. wässriges Ammoniak = 9:1:0.1)

Beispiel II

4-(Hexyloxycarbonylamidino)-nitrobenzol

Zu 3.5 g 4-Nitrobenzamidin-hydrochlorid und 7.2 g Kaliumcarbonat in einem Gemisch aus 350 ml Tetrahydrofuran und 70 ml Wasser werden unter Eiskühlung 2.8 ml Chlorameisensäurehexylester in 80 ml Tetrahydrofuran zugetropft. Nach einstündigem Rühren im Eisbad wird über Nacht bei Raumtemperatur stehen gelassen. Die organische Phase wird abgetrennt, zweimal mit gesättigter Kochsalzlösung gewaschen, getrocknet und eingeengt.
Ausbeute: 5.1 g 100 % der Theorie,
$R_f$-Wert: 0.72 (Kieselgel; Cyclohexan/Essigester = 1:1)
Analog Beispiel II werden folgende Verbindungen erhalten:

(1) 4-(Octyloxycarbonylamidino)-nitrobenzol
Massenspektrum: $M^+$ = 321

(2) 4-(Methoxycarbonylamidino)-nitrobenzol
Schmelzpunkt: 183-185°C

(3) 4-(Benzyloxycarbonylamidino)-nitrobenzol
$R_f$ Wert: 0.88 (Kieselgel; Methylenchlorid/Methanol/konz. wässriges Ammoniak = 9:1:0.1)

Beispiel III

4-[2-(Chlorcarbonyl)ethyl]-1-[(ethoxycarbonyl)methyl]-piperidin-hydrochlorid

Zu 1.46 g 4-(2-Carboxyethyl)-1-[(ethoxycarbonyl)methyl]-piperidin in 10 ml Methylenchlorid wird unter Rühren 1 ml gesättigte etherische Salzsäure zugegeben. Es werden 1.2 g Thionylchlorid hinzugefügt und 3 Stunden bei Raumtemperatur gerührt. Das Reaktionsgemisch wird eingeengt und der Rückstand zweimal mit Toluol versetzt und jeweils wieder eingeengt. Das Rohprodukt wird ohne Reinigung in Beispiel 1 weiter umgesetzt.
Analog Beispiel III werden folgende Verbindungen erhalten:

(1) 1-[2-(Chlorcarbonyl)ethyl]-4-[(methoxycarbonyl)methyl]-piperidin-hydrochlorid

(2) 4-[2-(Chlorcarbonyl)ethyl]-1-[(cyclohexyloxycarbonyl)-methyl]-piperidin-hydrochlorid

(3) 4-[2-(Chlorcarbonyl)ethyl]-1-[(isopropoxycarbonyl)-methyl]-piperidin-hydrochlorid

Beispiel IV

4-[2-(Carboxy)ethyl]-1-[(ethoxycarbonyl)methyl]-piperidin

10 g 4-[2-(Benzyloxycarbonyl)ethyl]-1-[(ethoxycarbonyl)methyl]-piperidin werden in 150 ml Tetrahydrofuran 4 Stunden bei Raumtemperatur und einem Wasserstoffdruck von 50 psi in Gegenwart von 1.3 g Palladium auf Aktivkohle hydriert. Das Reaktionsgemisch eingeengt und mit Diethylether und wenig Aceton kristallisiert.
Ausbeute: 5.8 g (79 % der Theorie),
Schmelzpunkt: 65-67°C
Analog Beispiel IV werden folgende Verbindungen erhalten:

(1) 4-(2-Carboxyethyl)-1-[(cyclohexyloxycarbonyl)methyl]-piperidin
Schmelzpunkt: 85-88°C

(2) 4-(2-Carboxyethyl)-1-[(isopropoxycarbonyl)methyl]-piperidin
$R_f$-Wert: 0.41 (Reversed Phase Kieselgel; Methanol/5%ige Kochsalzlösung = 6:4)

(3) 4-(2-Carboxyethyl)-1-[(methoxycarbonyl)methyl]-piperidin
Schmelzpunkt: 82-83°C

Beispiel V

4-[2-(Benzyloxycarbonyl)ethyl]-1-[(ethoxycarbonyl)methyl]-piperidin

Zu 9.0 g 4-[2-(Benzyloxycarbonyl)ethyl]-piperidin und 5,2 g N-Ethyl-diisopropylamin in 70 ml Acetonitril werden unter Rühren im Eisbad 6.35 g Bromessigsäureethylester in 20 ml Acetonitril zugetropft und 18 Stunden bei Raumtemperatur gerührt. Das Reaktionsgemisch wird eingeengt und der Rückstand rasch zwischen tert.Butyl-methylether, Eiswasser und 10 ml 2N Natronlauge verteilt. Die organische Phase wird abgetrennt mit Eiswasser und gesättigter Kochsalzlösung gewaschen, getrocknet und eingeengt.
Ausbeute: 10.05 g (83 % der Theorie),
$R_f$-Wert: 0.84 (Kieselgel; Methylenchlorid/Methanol/konz. wässriges Ammoniak = 95:5:1)
Analog Beispiel V werden folgende Verbindungen erhalten:

(1) 4-[2-(Benzyloxycarbonyl)ethyl]-1-[(cyclohexyloxycarbonyl)-methyl]-piperidin
$R_f$-Wert: 0.47 (Kieselgel; Methylenchlorid/Methanol/konz. wäss riges Ammoniak = 98:2:0,5).

(2) 4-[2-(Benzyloxycarbonyl)ethyl]-1-[(isopropoxycarbonyl)-methyl]-piperidin
Massenspektrum: $M^+$ = 347

(3) 4-[2-(Benzyloxycarbonyl)ethyl]-1-[(methoxycarbonyl)-methyl]-piperidin
$R_f$-Wert: 0.40 (Kieselgel; Methylenchlorid/Methanol/konz. wäss riges Ammoniak = 9:1:0,1)

Beispiel VI

4-[2-(Benzyloxycarbonyl)ethyl]-piperidin

9,7 g 4-(2-Carboxyethyl)piperidin-hydrochlorid (Schmelzpunkt: 240-250°C, hergestellt durch Hydrierung von 3-(4-Pyridyl)-acrylsäure in Eisessig in Gegenwart von Platinoxid und anschließender Behandlung mit Salzsäure), 30 ml Benzylalkohol, 3 g p-Toluolsulfonsäure und 50 ml Toluol werden 75 Minuten am Wasserabscheider erhitzt. Das Reaktionsgemisch wird im Vakuum eingeengt, der Rückstand mit 50 ml Eiswasser versetzt und dreimal mit tert.Butyl-methylether extrahiert. Die wässrige Phase wird alkalisch gestellt und mit tert.Butyl-methylether extrahiert. Der Extrakt wird mit Kochsalzlösung gewaschen, getrocknet und eingeengt.
Ausbeute: 9.0 g (73 % der Theorie),
$R_f$-Wert: 0.18 (Kieselgel; Methylenchlorid/Methanol/konz. wässriges Ammoniak = 95:5:1)

Herstellung der Endverbindungen:

Beispiel 1

4-[2-[[4-(Octyloxycarbonylamidino)phenyl]aminocarbonyl]ethyl]-1-[(ethoxycarbonyl)methyl]-piperidin

Zu 5.1 g 4-(Octyloxycarbonylamidino)anilin und 100 mg 4-Dimethylaminopyridin in 30 ml Pyridin werden unter Eiskühlung 5.7 g 4-[2-(Chlorcarbonyl)ethyl]-1-[(ethoxycarbonyl)methylpiperidin-hydrochlorid in 30 ml Methylenchlorid innerhalb von 30 Minuten zugetropft. Nach Stehen bei Raumtemperatur über Nacht wird das Reaktionsgemisch eingeengt und der Rückstand durch Chromatographie über eine Kieselgelsäule mit Methylenchlorid/Methanol/konz. wässriges Ammoniak (9:1:0.1) gereinigt. Ausbeute: 2.9 g (32 % der Theorie),
Schmelzpunkt: 151-153°C
Massenspektrum: $(M+H)^+$ = 517
Analog Beispiel 1 werden folgende Verbindungen erhalten:

(1) 4-[2-[[4-(Hexyloxycarbonylamidino)phenyl]aminocarbonyl]-ethyl]-1-[(ethoxycarbonyl)methyl]-piperidin
Schmelzpunkt: 151-153°C Massenspektrum: $M^+$ = 489

(2) 4-[2-[[4-(Hexyloxycarbonylamidino)phenyl]aminocarbonyl]-ethyl]-1-[(isopropoxycarbonyl)methyl]-piperidin
Schmelzpunkt: 161-162°C
$R_f$-Wert: 0,20 (Kieselgel; Methylenchlorid/Methanol/konz. wässriges Ammoniak = 9:1:0.1)

(3) 4-[2-[[4-(Octyloxycarbonylamidino)phenyl]aminocarbonyl]-ethyl]-1-[(isopropoxycarbonyl)methyl]-piperidin

Schmelzpunkt: 151-152°C
Massenspektrum: $M^+$ = 531

(4) 4-[2-[[4-(Hexyloxycarbonylamidino)phenyl]aminocarbonyl]ethyl]-1-[(cyclohexyloxycarbonyl)methyl]-piperidin
Schmelzpunkt: 149-151°C Massenspektrum: $(M+H)^+$ = 543

(5) 4-[2-[[4-(Octyloxycarbonylamidino)phenyl]aminocarbonyl]ethyl]-1-[(cyclohexyloxycarbonyl)methyl]-piperidin
Schmelzpunkt: 157-162°C
Massenspektrum: $(M+H)^+$ = 571

(6) 4-[2-[[4-(Hexyloxycarbonylamidino)phenyl]aminocarbonyl]ethyl]-1-[(methoxycarbonyl)methyl]-piperidin
Schmelzpunkt: 153-155°C
$R_f$-Wert: 0.32 (Kieselgel; Methylenchlorid/Methanol/konz. wässriges Ammoniak = 9:1:0.1)

(7) 4-[2-[[4-(Octyloxycarbonylamidino)phenyl]aminocarbonyl]ethyl]-1-[(methoxycarbonyl)methyl]-piperidin
Schmelzpunkt: 149-150°C
Massenspektrum: $(M+H)^+$ = 503

(8) 4-[2-[[4-(Decyloxycarbonylamidino) phenyl]aminocarbonyl]ethyl]-1-[(ethoxycarbonyl)methyl]-piperidin

(9) 4-[2-[[4-(Dodecyloxycarbonylamidino)phenyl] aminocarbonyl]-ethyl]-1-[(ethoxycarbonyl)methyl]-piperidin

(10) 4-[2-[[4-(Tetradecyloxycarbonylamidino)phenyl]aminocarbonyl]-ethyl]-1-[(ethoxycarbonyl)methyl]-piperidin

(11) 4-[2-[[4-(Hexadecyloxycarbonylamidino)phenyl]aminocarbonyl]-ethyl]-1-[(ethoxycarbonyl)methyl]-piperidin

(12) 4-[2-[[4-(Octadecyloxycarbonylamidino)phenyl]aminocarbonyl]-ethyl]-1-[(ethoxycarbonyl)methyl]-piperidin

Beispiel 2

4-[2-[[4-(Hexyloxycarbonylamidino)phenyl]aminocarbonyl]-ethyl]-1-[(ethoxycarbonyl)methyl]-piperidin-methansulfonat

Zu 600 mg 4-[2-[[4-(Hexyloxycarbonylamidino)phenyl]aminocarbonyl]-ethyl]-1-[(ethoxycarbonyl)methyl]-piperidin in 30 ml Aceton werden 1,228 ml einer 1-molaren Lösung von Methansulfonsäure in Aceton getropft. Nach Stehen über Nacht und Anreiben mit einem Glasstab wird ein Feststoff erhalten, der abgesaugt und zweimal mit Aceton gewaschen wird. Anschließend wird in Vakuum bei 40°C getrocknet.
Ausbeute: 560 mg (78 % der Theorie),
Schmelzpunkt: 117-120°C

| | | | | | | | | |
|---|---|---|---|---|---|---|---|---|
| Ber. | C | 55.46 | H | 7.58 | N | 9.58 | S | 5.48 |
| Gef. | | 55.56 | | 7.85 | | 9.72 | | 5.54 |

Analog Beispiel 2 wird folgende Verbindung erhalten:
(1) 4-[2-[[4-(Octyloxycarbonylamidino)phenyl]aminocarbonyl]-ethyl]-1-[(ethoxycarbonyl)methyl]-piperidin-methansulfonat
Schmelzpunkt: 125-127°C

| | | | | | | | | |
|---|---|---|---|---|---|---|---|---|
| Ber. | C | 56.84 | H | 7.90 | N | 9.14 | S | 5.23 |
| Gef. | | 56.94 | | 7.88 | | 9.32 | | 5.27 |

Beispiel 3

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

Beispiel 4

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

Beispiel 5

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 6

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

1. Phenylamidine der allgemeinen Formel

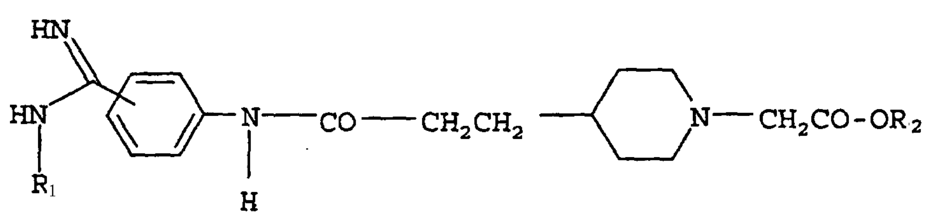

(I),

in der
$R_1$ eine $C_{5-12}$-Alkyloxycarbonyl- und
$R_2$ eine $C_{1-4}$-Alkyl- oder $C_{5-6}$-Cycloalkylgruppe bedeuten,
deren Tautomere, deren Stereoisomere und deren Salze.

2. Phenylamidine der allgemeinen Formel I gemäß Anspruch 1, in denen die substituierte Amidinogruppe in 4-Stellung steht,
deren Tautomere, deren Stereoisomere und deren Salze.

3. Folgende Phenylamidine der allgemeinen Formel I gemäß Anspruch 1:

(1) 4-[2-[[4-(Octyloxycarbonylamidino)phenyl]aminocarbonyl]-ethyl]-1-[(ethoxycarbonyl)methyl]-piperidin,

(2) 4-[2-[[4-(Hexyloxycarbonylamidino)phenyl]aminocarbonyl]-ethyl]-1-[(ethoxycarbonyl)methyl]-piperidin,

(3) 4-[2-[[4-(Hexyloxycarbonylamidino)phenyl]aminocarbonyl]-ethyl]-1-[(methoxycarbonyl)methyl]-piperidin und

(4) 4-[2-[[4-(Octyloxycarbonylamidino) phenyl] aminocarbonyl]-ethyl]-1-[(methoxycarbonyl)methyl]-piperidin

sowie deren Salze.

4. Physiologisch verträgliche Salze der Verbindungen nach mindestens einem der Ansprüche 1 bis 3 mit anorganischen oder organischen Säuren oder Basen.

5. Arzneimittel, enthaltend eine Verbindung nach mindestens einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln.

6. Verwendung einer Verbindung nach mindestens einem der Ansprüche 1 bis 4 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

7. Verfahren zur Herstellung eines Arzneimittels gemäß Anspruch 5, **dadurch gekennzeichnet, daß** auf nicht chemischem Wege eine Verbindung nach mindestens einem der Ansprüche 1 bis 4 in einen oder mehrere inerte Trägerstoffe und/oder Verdünnungsmittel eingearbeitet wird.

8. Verfahren zur Herstellung der Verbindungen der allgemeinen Formel I gemäß den Ansprüchen 1 bis 4, **dadurch gekennzeichnet, daß** eine Verbindung der allgemeinen Formel

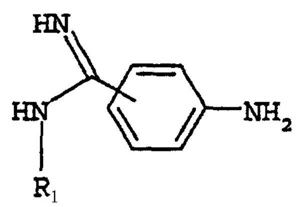

(II),

in der
$R_1$ wie in den Ansprüchen 1 bis 3 definiert ist, mit einer Verbindung der allgemeinen Formel

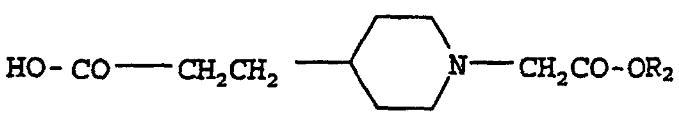

(III),

in der
$R_2$ wie in den Ansprüchen 1 bis 3 definiert ist, oder mit deren reaktionsfähigen Derivaten umgesetzt und gegebenenfalls anschließend der Rest $R_7$ in ein Wasserstoffatom übergeführt wird und
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträgliche Salze übergeführt wird.

## Claims

1. Phenylamidines of general formula

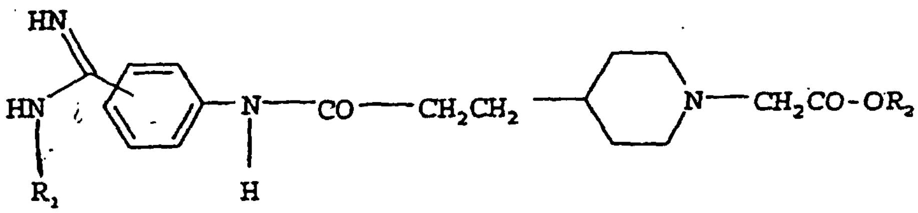

(I)

wherein
$R_1$ denotes a $C_{5-12}$-alkyloxycarbonyl and
$R_2$ denotes a $C_{1-4}$-alkyl or $C_{5-6}$-cycloalkyl group,
the tautomers, stereoisomers and salts thereof.

2. Phenylamidines of general formula I according to claim 1, wherein the substituted amidino group is in the 4 position, the tautomers, stereoisomers and salts thereof.

3. The following phenylamidines of general formula I according to claim 1:

(1) 4-[2-[[4-(octyloxycarbonylamidino)phenyl]-aminocarbonyl]ethyl]-1-[(ethoxycarbonyl)methyl]-piperidine,

(2) 4-[2-[[4-(hexyloxycarbonylamidino)phenyl]-aminocarbonyl]-ethyl]-1-[(ethoxycarbonyl)methyl]-piperidine,

(3) 4-[2-[[4-(hexyloxycarbonylamidino)phenyl]-aminocarbonyl]ethyl]-1-[(methoxycarbonyl)methyl]-piperidine and

(4) 4-[2-[[4-(octyloxycarbonylamidino)phenyl]-aminocarbonyl]ethyl]-1-[(methoxycarbonyl)methyl]-piperidine

and the salts thereof.

4. Physiologically acceptable salts of the compounds according to at least one of the claims 1 to 3 with inorganic or organic acids or bases.

5. Pharmaceutical compositions containing a compound according to at least one of claims 1 to 3 or a physiologically acceptable salt according to claim 4 optionally together with one or more inert carriers and/or diluents.

6. Use of a compound according to at least one of claims 1 to 4 for preparing a pharmaceutical composition which is suitable for fighting or preventing diseases in which smaller or larger cell aggregations occur or cell-matrix interactions are involved.

7. Process for preparing a pharmaceutical composition according to claim 5, **characterised in that** a compound according to at least one of claims 1 to 4 is incorporated in one or more inert carriers and/or diluents by a non-chemical method.

8. Process for preparing the compounds of general formula I according to claims 1 to 4, **characterised in that** a compound of general formula

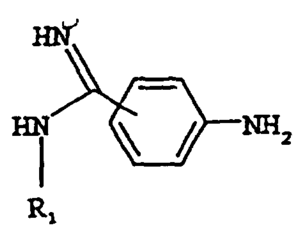

(II),

wherein

$R_1$ is defined as in claims 1 to 3, is reacted with a compound of general formula

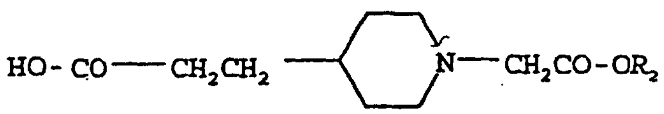

(III),

wherein
$R_2$ is defined as in claims 1 to 3, or a reactive derivative thereof, and
the group $R_2$ is optionally subsequently converted into a hydrogen atom
and
if desired a compound of general formula I thus obtained is resolved into its stereoisomers and/or
a compound of general formula I thus obtained is converted into its salts, particularly, for pharmaceutical use, into its physiologically acceptable salts.

## Revendications

1. Phénylamidines de formule générale

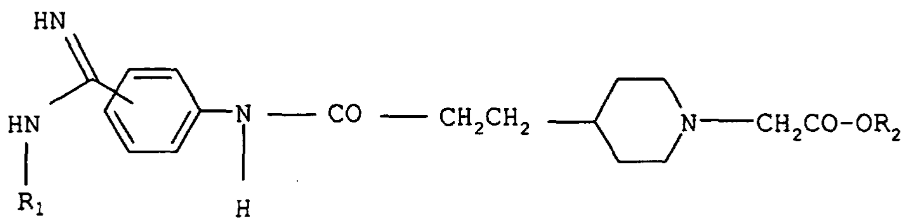

(I),

dans laquelle
$R_1$ désigne un groupe alkyloxycarbonyle en $C_5$ à $C_{12}$ et
$R_2$ désigne un groupe alkyle en $C_1$ à $C_4$ ou un groupe cycloalkyle en $C_5$ à $C_6$, leurs tautomères, leurs stéréoisomères et leurs sels.

2. Phénylamidines de formule générale I selon la revendication 1, dans lesquelles le groupe amidine substitué se trouve en position 4, leurs tautomères, leurs stéréoisomères et leurs sels.

3. Les phénylamidines suivantes de formule générale I selon la revendication 1 :

(1) 4-[2-[[4-(octyloxycarbonylamidino)phényl]aminocarbonyl]-éthyl-1-[(éthoxycarbonyl)méthyl]-pipéridine,
(2) 4-[2-[[4-(hexyloxycarbonylamidino)phényl]aminocarbonyl]-éthyl-1-[(éthoxycarbonyl)méthyl]-pipéridine,
(3) 4-[2-[[4-(hexyloxycarbonylamidino)phényl]aminocarbonyl]-éthyl]-1-[(méthoxycarbonyl)méthyl]-pipéridine et
(4) 4-[2-[[4-(octyloxycarbonylamidino)phényl]aminocarbonyl]-éthyl-1-[(méthoxycarbonyl)méthyl]-pipéridine

ainsi que leurs sels.

4. Sels des composés compatibles d'un point de vue physiologique selon au moins l'une des revendications 1 à 3 avec des acides ou des bases inorganiques ou organiques.

5. Produit pharmaceutique, contenant un composé selon au moins l'une des revendications 1 à 3 ou un sel compatible d'un point de vue physiologique selon la revendication 4 avec en outre, le cas échéant, un ou plusieurs supports inertes et / ou un ou plusieurs diluants.

6. Utilisation d'un composé selon au moins l'une des revendications 1 à 4 pour la fabrication d'un produit pharmaceutique, qui convient dans la lutte ou la prévention des maladies, au sein desquelles des agrégats cellulaires de plus petite taille ou de plus grande taille surviennent ou dans lesquelles des interactions matricielles entre les

cellules jouent un rôle.

**7.** Procédé de fabrication d'un produit pharmaceutique selon la revendication 5 **caractérisé en ce qu'**un composé selon au moins l'une des revendications 1 à 4 est incorporé d'une manière non chimique dans un ou plusieurs supports inertes et / ou dans un ou plusieurs diluants.

**8.** Procédé de préparation de composés de formule générale I selon les revendications 1 à 4, **caractérisé en ce qu'**on fait réagir un composé de formule générale

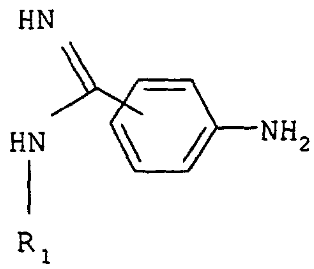

(II),

dans laquelle

$R_1$ est défini conformément aux revendications 1 à 3, avec un composé de formule générale

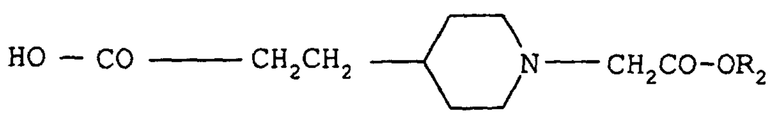

(III),

dans laquelle

$R_2$ est défini conformément aux revendications 1 à 3, ou est mis à réagir avec ses dérivés réactifs et,

le cas échéant, le reste de $R_2$ est par la suite transformé en un atome d'hydrogène et

si souhaité, un composé ainsi obtenu, de formule générale I, est séparé de son stéréosimère et / ou un composé ainsi obtenu, de formule générale I, est transformée en ses sels, et notamment dans le cadre d'applications pharmaceutiques, en sels compatibles d'un point de vue physiologique.